# EUROPÄISCHE PATENTSCHRIFT

(11) **EP 1 397 216 B1**
(45) Veröffentlichungstag und Bekanntmachung des Hinweises auf die Patenterteilung: **22.08.2007**
(21) Anmeldenummer: 02724299.9
(22) Anmeldetag: 26.04.2002
(51) Int. Cl.: B02C 15/00

(54) **VERFAHREN ZUR PARALLELEN SYNTHESE UND ÜBERTRAGUNG VON MOLEKÜLEN AUF EIN SUBSTRAT**
METHOD FOR PARALLEL SYNTHESIS AND TRANSFER OF MOLECULES TO A SUBSTRATE
PROCEDE DE SYNTHESE ET DE TRANSFERT PARALLELES DE MOLECULES SUR UN SUBSTRAT

(30) Priorität: 26.04.2001 DE 10121574; 04.05.2001 DE 10123644
(43) Veröffentlichungstag der Anmeldung: 17.03.2004
(73) Patentinhaber: Indigon GmbH, 72070 Tübingen (DE)
(72) Erfinder: BERNARD, André, 72020 Tübingen (DE); DÜBEL, Stefan, 69221 Dossenheim (DE)
(74) Vertreter: Mütschele, Thomas
(86) Internationale Anmeldenummer: PCT/EP2002/004670
(87) Internationale Veröffentlichungsnummer: WO 2002/087769

(56) Entgegenhaltungen:
- DE-A- 19 543 232
- US-A- 6 096 386

## Beschreibung

Die vorliegende Erfindung betrifft ein Verfahren zur hochparallelen Herstellung von Molekül-Bibliotheken auf einer Substrat-Oberfläche.

Die Microarray-Technologie, bei der viele verschiedene Biomoleküle wie DNA oder Proteine dicht gepackt in einem vordefinierten Muster auf einer Substrat-Oberfläche aufgebracht sind, ist mittlerweile zur Standardmethode für die parallele Analyse biologischer Proben geworden. Diese Technologie wird z.B. bei der Analyse der Genexpression, bei der genetischen Diagnostik, in der biologischen und pharmazeutischen Forschung und zur Bestimmung genmanipulierter Organismen in der Lebensmittelindustrie verwendet.

Für eine Massenproduktion von Molekül-Arrays ist das vielfach reproduzierbare Aufbringen von Molekülen auf Metall-, Glas-, Membran-oder Kunststoff-Oberflächen der limitierende Kosten- und Zeitfaktor. Bei diesem Verfahren sind prinzipiell die folgenden zwei Techniken bekannt: 1) eine in situ-Synthese von Array-Molekülen aus Monomeren mittels photochemisch- oder elektrostatisch-vermittelter Reaktionen am Ort direkt auf einem Träger (US 5,405,783); 2) ein Auftragen von fertigen Makromolekülen entweder in Tropfenform *(spotting)* mittels Drucknadel (US 6,101,946), Mikropipetten (US 5,601,980) oder Tintenstrahldruckern (US 5,927,547). Zum gegenwärtigen Stand der Technik, insbesondere zur Verwendung von Microarrays bei der DNA- und Protein-Analyse, wird auf folgende einschlägige Übersichtsartikel verwiesen: Nature Genetics, Vol. 21, No.1 supplement (1999).

Beim Auftragen von Makromolekülen auf die Träger-Oberfläche wird der Durchmesser der Molekül-Fixierstellen auf dem Träger (Spots) durch die . Beschaffenheit der Substrat-Oberfläche und die Tropfengröße bzw. die Größe der Drucknadel bestimmt, mit der die Moleküle aufgetragen werden. Typischerweise betragen die Spot-Größen bei den bekannten Verfahren 50 µm bis 200 µm. Nur durch die Anwendung der photolithographischen Technik, die speziell dafür hergestellte Lichtmasken zu einer direkten Festphasen-Synthese der DNA vor Ort verwendet (WO 92/10092), kann eine wesentlich höhere Dichte und kleinere Spot-Größen erreicht werden. Dieses Verfahren ist allerdings mit einem erheblichen technischen sowie Zeit- und Kostenaufwand verbunden.

Die bekannten Verfahren zur Herstellung von Molekül-Arrays besitzen den Nachteil, daß viele Schritte für die Herstellung jedes einzelnen Arrays benötigt werden, so dass keine oder nur geringe Parallelisierung des Prozesses möglich ist Die unterschiedlichen Molekül-Sorten müssen jeweils einzeln auf die Array-Oberfläche aufgebracht werden, das bei vielen Tausenden Spots pro Array immens viele Einzelschritte zur Folge hat. Das ist sehr zeit-, geräte- und kostenintensiv und schafft Probleme bei der Reproduzierbarkeit.

Ziel der vorliegenden Erfindung ist es, ein Verfahren zu entwickeln, das eine schnellere und kostengünstigere Herstellung von Molekül-Bibliotheken auf einer Substrat-Oberfläche mit höherer Präzision und Reproduzierbarkeit erlaubt. Zudem soll es mit diesem Verfahren möglich sein, eine Spot-Größe von weniger als 10 µm, und im Besonderen weniger als 2 µm, sowie eine höhere Spot-Dichte als durch Standardverfahren wie beispielsweise Tropfenapplikation zu erreichen.

Zur Lösung dieser Aufgabe schlägt die Erfindung ein Verfahren mit den im Anspruch 1 genannten Merkmalen vor. Weiterbildungen der Erfindung sind Gegenstand der übrigen abhängigen Ansprüche 2 bis 28, deren Wortlaut ebenso wie der Wortlaut der Zusammenfassung durch Bezugnahme zum Inhalt der Beschreibung gemacht wird. Durch die Verwendung des erfindungsgemäßen Verfahrens wird die Herstellung von Molekül-Arrays stark parallelisiert und der Produktionsprozeß vereinfacht und beschleunigt.

Die Erfindung beschreibt eine Methode zur ortsaufgelösten und selektiven Synthese komplexer chemischer Verbindungen auf einer Substrat-Oberfläche, wobei vorzugsweise deren Gesamtheit eine Punktmatrix, eine kreisförmige, spiralförmige, streifenförmige, lineare oder sonstige geometrische oder stochastische Anordnung von Molekülen auf einer Substrat-Oberfläche bildet.

Das oben genannte Ziel wird gemäß der Erfindung dadurch erreicht, daß zum einen eine Stempeltechnik verwendet wird, mit der die gesamte Substrat-Oberfläche oder Teilflächen in einem einzigen Druckvorgang mit verschiedenen Biomolekülen beschichtet werden. Zum anderen werden diese Biomoleküle anhand einer auf dem Stempel aufgebrachten Matrize (*template*) jeweils zwischen den Stempelvorgängen auf dem Substrat ortstreu neu synthetisiert. Durch die Neusynthese von Array-Molekülen auf der Oberfläche des Stempels erfolgt seine Regeneration für den neuen Druckvorgang. Der Stempel dient somit nicht nur als Transportvehikel zur Übertragung der Array-Moleküle an den Bestimmungsort auf der Substrat-Oberfläche, sondern stellt auch einen Bauplan für eine reproduzierbare Synthese dieser Moleküle dar.

Als Stempelmaterial wird vorteilhaft Kunststoff, Glas oder Metall verwendet. In einer bevorzugten Ausführung der Erfindung wird ein Polymer mit elastischen 5 Eigenschaften (Elastomer) oder eine Kombination verschiedener Materialien, insbesondere ein Stempel mit einer Oberflächenschicht aus einem Elastomer und einem Kern aus einem anderen Stoff verwendet. In einer besonders bevorzugten Ausführung wird Polydimethylsiloxan (PDMS) für die Stempel-Herstellung verwendet. PDMS ist ein Kunststoff, der in beliebige Formen gegossen und gehärtet werden kann (Delamarche et al., Advanced Materials 9: 741-746 (1997)). Es eignet sich als Stempelmaterial besonders gut, da es aufgrund seiner physikalischen Eigenschaften in der Lage ist, sich der zu bestempelnden Oberfläche höchst genau anzupassen, und somit einen gleichmäßigen Kontakt beider Oberflächen zu gewährleisten.

Man bedient sich erfindungsgemäß eines Stempels, der ein Array von Matrizen-Molekülen auf seiner Oberfläche trägt. Die Matrizen-Moleküle können kovalent oder nichtkovalent mit der Stempel-Oberfläche verbunden oder auf der Stempel- Oberfläche aufgetragen sein. Das Aufbringen der Matrizen-Moleküle auf den Stempel kann mechanisch nach Stand der Technik insbesondere durch eine Tropfenapplikation 20 z.B. mit Hilfe von Tintenstrahldruckern oder Drucknadeln, oder mittels lithographischer Methoden nach Stand der Technik geschehen. Das Beladen des Stempels mit den Matrizen-Molekülen kann alternativ auch in einem oder mehreren Stempelvorgängen erfolgen, bei denen jeweils einzelne Molekül-Bestandteile durch eine stufenweise Synthese zu kompletten Makromolekülen zusammengebaut werden. Möglich ist es auch, verschiedene komplette Moleküle durch einen kongruenten Satz mehrerer Stempel oder Masken an vordefinierten Positionen auf der Stempel-Oberfläche aufzubringen.

In einer anderen vorteilhaften Variante wird ein für eine bestimmte Lichtwellenlänge transparentes Material wie beispielsweise Polydimethylsiloxan für die Stempelherstellung verwendet. Die lichtleitenden Eigenschaften des Stempels können erfindungsgemäß dazu verwendet werden, um über die ortsselektive Lichtleitung bestimmte Moleküle an den vordefinierten Orten anzukoppeln bzw. zu synthetisieren.

Ebenso ist eine durch elektrische Felder gesteuerte Synthese direkt auf dem Stempel denkbar.

Die kovalente Verknüpfung der Matrizen-Moleküle kann beispielsweise über eine Bindung bereits vorhandener oder speziell eingeführter amino-, thio- oder phospho-Gruppen an eine endgruppen-funktionalisierte silanisierte Oberfläche erreicht werden. Alternativ können biotinylierte Matrizen-Moleküle mittels Streptavidin-Beschichtung auf der Stempel-Oberfläche spezifisch immobilisiert werden.

An der Matrize auf der Stempel-Oberfläche werden in einer heterogenen Synthese Abbild-Moleküle (*image molecules*) enzymatisch nach einer Standardtechnik hergestellt. Die Abbild-Moleküle verbleiben nach der Synthese am Ort der jeweiligen Matrizen-Moleküle auf dem Stempel. Die Abbild-Moleküle können erfindungsgemäß einen Anker enthalten, mit dem sie an einem Bestimmungsort auf der Ziel-Oberfläche immobilisiert werden können.

In einer Ausführungsvariante kann als Anker beispielsweise ein Oligopeptid ("Tag") dienen. Die zu bestempelnde Oberfläche wird in diesem Fall in Vorbereitung des Stempelvorganges mit einer Schicht von Molekülen versehen, die mit hoher Affinität an die "Tag"-Gruppe der neu synthetisierten Abbild-Moleküle binden. Diese Beschichtung kann ebenfalls durch Stempeln aufgebracht werden und auf bestimmte Bereiche der Oberfläche beschränkt sein, um Positionsinformation für den späteren Analyse- und Auslesevorgang zu erzeugen. Das "Tag" kann im Besonderen aus fünfzehn bis zwanzig N-terminalen Aminosäuren der humanen pankreatischen Rnase bestehen, die mit hoher Affinität an ein proteolytisches Spaltstück der homologen Kuh-RNase oder ein entsprechendes Fragment aus anderen Spezies bindet. In einer weiteren typischen Ausführung ist dieses "Tag" das Epitop-Peptid eines Antikörpers oder eines anderen Moleküls, z.B. des Streptavidins. Alternativ kann z.B. Biotin an die Abbild-Moleküle gekoppelt werden, welches eine hochaffine Bindung an das mit Streptavidin oder Avidin beschichtete Substrat bewirkt. Andere Haptene und ihre Bindeproteine wie PHOX (4-Ethoxymethylene-2-Phenyl-2-Oxazoline-5-one), Digoxigenin, FITC u.a. können ebenfalls verwendet werden.

Der mit Abbild-Molekülen beladene Matrizen-Stempel wird mit der Ziel-Oberfläche in Kontakt gebracht. In einem Kontaktdruck-Verfahren werden die frisch synthetisierten Abbild-Moleküle der einzelnen Spots übertragen. Auf dem Stempel bleiben die Matrizen-Moleküle zurück, die für eine erneute Synthese der Abbild-Moleküle auf der Stempel-Oberfläche wieder zur Verfügung stehen. Nach einer StempelRegeneration kann ein weiteres Substrat bestempelt werden.

Das erfindungsgemäße Verfahren besitzt gegenüber den bekannten Verfahren den Vorteil, daß mehrere unterschiedliche Molekülsorten in einem Vorgang auf die Substrat-Oberfläche übertragen werden, und somit jedes neue Array in jeweils einem einzigen Schritt hergestellt werden kann. Diese Abfolge von Vorgängen, - Stempelregeneration durch die Neusynthese, Stempeln, - kann erfindungsgemäß mehrfach wiederholt werden. Die eigentliche Herstellung und Gestaltung des Matrizen-Arrays, und das Aufbringen der Matrizen-Moleküle auf die Stempel- Oberfläche wird bei dem hier beschriebenen Verfahren nur ein einziges Mal ausgeführt. Der fertige Stempel dient dann als Vorlage zur Synthese mehrerer Abbild-Arrays.

In einer weiteren Ausführung der Erfindung kann die beschriebene Methode dahingehend erweitert werden, daß auf einem anderen Weg, z.B. in einem Standardverfahren hergestellte Arrays (im Weiteren Original-Arrays genannt), kopiert und vervielfältigt werden können. Dazu wird das Original-Array mit darauf platzierten Array-Molekülen als Vorlage zur Synthese komplementärer Moleküle benutzt, die in weiteren Schritten als Matrize zur Synthese von Abbild-Molekülen dienen. Diese Matrizen-Moleküle werden von einem noch leeren Stempel aufgenommen und gebunden. Anker-Elemente, die an den neu synthetisierten Matrizen-Molekülen angebracht werden, können deren Anbindung an die Stempel-Oberfläche begünstigen. Die Matrizen-Moleküle auf dem Stempel können für die Schritte der wiederholbaren Abbild-Synthese und des Stempelns auf die Ziel-Oberfläche wie oben beschrieben verwendet werden.

Ausgehend von einem nach dem beschriebenen Verfahren hergestellten Abbild-Array kann ein oder mehrere weitere Matrizen-Stempel generiert werden, die wiederum für die Synthese von Abbild-Arrays eingesetzt werden. Über derartige iterative Synthese von Matrizen- und Abbild-Arrays kann eine erhebliche Vervielfältigung der Matrizen-Stempel erreicht werden, die in einem Produktionsprozeß bei der industriellen 30 Herstellung einer großen Zahl von Molekül-Arrays verwendet werden können.

Die Erfindung ist mit einer Vielzahl weiterer Vorteile verbunden. So ist das Verfahren nicht auf die Synthese kurzer Oligomere beschränkt wie andere bekannte Verfahren. Die Erfindung ermöglicht das Aufbringen hochkomplexer Substanzen und sogar von Molekülkomplexen inklusive Modifikationen auf ein Substrat. Außerdem können Arrays mit mehreren Spots, insbesondere mit mehr als 1000, in einer definierbaren räumlichen Verteilung in einem einzigen Schritt hergestellt werden. Dabei kann die genaue Position jedes einzelnen Molekül-Spots über seine relative Anordnung zu einer Markierung auf dem Substrat leicht ermittelt werden.

Des weiteren kann die beschriebene Methode vorteilhaft dazu verwendet werden, um Arrays mit kleineren Spot-Größen sowie höheren Spot-Dichten als durch bekannte Verfahren, z.B. durch eine Tropfenapplikation, herzustellen. Dies kann erreicht werden, indem auf dem Matrizen-Stempel neben der chemischen Information in Form von Matrizen-Molekülen, die z.B. durch Tropfenapplikation mit einer definierten Spot-Dichte auf die Stempel-Oberfläche aufgetragen werden, zusätzlich eine topologische Information in Form von Relief-Strukturen vorhanden ist. Die topologischen Strukturen können z.B. Erhebungen sein, deren Dimensionen kleiner als 1 µm sein können. Ein konformaler Kontakt zur Substrat-Oberfläche ist die Grundvoraussetzung für die Vewendung des Stempels im erfindungsgemäßen Verfahren. Zusätzlich dürfen die Strukturelemente beim Kontakt zur Substrat-Oberfläche nicht durchhängen und sich nicht zu stark verformen. Zur Charakterisierung der Strukturen dienen hier der Formfaktor (*aspect ratio:* Verhältnis der Strukturhöhe zu ihren lateralen Dimensionen bei periodischen Strukturen) und der Füllfaktor (*filling factor*: Verhältnis der Kontaktfläche der Strukturen zur Gesamtfläche) (s. auch: Delamarche et al., Advanced Materials 9: 741-746 (1997); Bietsch and Michel, J. Appl. Phys. 88: 4310-4318 (2000)). Ein Formalfaktor von 1:5 bis 5:1, in einigen Fällen bei Verwendung geeigneter Materialien von 1:20 bis 20:1, ist besonders empfehlenswert. Der Füllfaktor soll mindestens 5 % - 10 % betragen.

Die Querschnittsfläche der Erhebungen wird vorzugsweise kleiner als die Fläche der nach Stand der Technik durch eine Tropfenapplikation generierten Spots gefertigt. In einer bevorzugten Ausführung ist der Abstand zwischen benachbarten Erhebungen gleich dem Abstand zwischen einzelnen benachbarten Spots auf dem Stempel, so daß die Spots im selben Raster wie die jeweiligen Erhebungen positioniert werden. Die durch das Aufbringungsverfahren normalerweise unscharfen Spot-Ränder reichen über die Kanten der Erhebungen. Nach einer Synthese werden beim Stempeln jedoch nur die auf der hervorstehenden proximalen glatten Fläche positionierten Abbild-Moleküle übertragen, da nur sie mit der Substrat-Oberfläche in direkten Kontakt kommen.

Dadurch reduziert sich die Spot-Größe im Vergleich zu der nach einem Standardverfahren z.B. durch eine Tropfenapplikation erzeugten Spot-Größe. Um eine höhere Spot-Dichte als auf dem Stempel zu erreichen, werden mehrere Stempelvorgänge getätigt. Vor jedem neuen Stempelvorgang wird der Stempel durch eine Translation oder / und eine Rotation relativ zur Stempel-Oberfläche verschoben, so daß die neuen Spots zwischen den zuvor aufgetragenen Spots plaziert werden. Auf diese Weise lassen sich Spots in eine beliebige dreidimensionale Anordnung auf der Substrat-Oberfläche bringen.

Wenn beispielsweise Spots nach einem für Microarrays üblichen regulären Matrizen-Muster aufgetragen werden müssen, wird die Substrat-Oberfläche bei jedem nächsten Stempelvorgang um eine definierte Länge b, die kleiner als der Abstand a zwischen den Erhebungen auf dem Stempel ist, entlang ihrer x- oder y-Achse relativ zur Stempel-Oberfläche verschoben. Bei den aufeinanderfolgenden Stempelvorgängen werden dabei bevorzugt Stempel mit der gleichen Topologie aber mit unterschiedlichen Beschichtungen verwendet. Wenn der Abstand zwischen den benachbarten Spots auf dem Stempel a und die Dichte *D₁* beträgt, und ein Array mit einem Abstand zwischen den benachbarten Spots b und einer Dichte *D₂* hergestellt werden soll, so werden *a*₂/*b*₂ *(D₂*/*D₁)* verschiedene Stempel und Stempelvorgänge benötigt.

In einer anderen Variante kann eine kleinere Spot-Größe über die Einführung einer Reliefstruktur auf dem Substrat erzielt werden. Alternativ werden Reliefstrukturen auf beiden Oberflächen, dem Stempel und dem Substrat, aneinander so angepaßt, daß die gewünschte Spot-Dichte erzeugt wird.

In einer weiteren Ausführung kann das Matrizen-Array für den topologischen Stempel an Molekülen des Original-Arrays synthetisiert werden. Dabei werden Stempel verwendet, bei denen der Abstand zwischen den benachbarten Erhebungen gleich dem Abstand zwischen den benachbarten Spots auf dem Original-Array, ihre Querschnittsfläche jedoch kleiner als die Spot-Fläche ist. Auf diese Weise wird beim Stempeln die Spot-Größe reduziert. Durch ein versetztes Kontaktdrucken mit unterschiedlichen topologischen Matrizen-Stempeln lassen sich die Original-Arrays nicht nur kopieren, sondern auch um einige Größenordnungen komprimieren. Durch die Verwendung von Stempeln mit unregelmäßigen vordefinierten Relief-Strukturen können ferner aus einem Original-Array bestimmte gewünschte Molekül-Spots ausselektiert und zu einem neuen Abbild-Array zusammengesetzt werden.

Durch die Verwendung topologischer Stempel kann vorteilhaft eine Spot-Größe von weniger als 10 µm, und im Besonderen von weniger als 2 µm erreicht werden.

An Materialien für die Herstellung topologischer Stempel werden folgende Anforderungen gestellt: präzise Strukturierbarkeit, abdichtender Kontakt zu einem geeigneten Substrat *(conformal contact),* chemische und physikalische Resistenz gegenüber Reaktions-Bedingungen. Ferner muß bei kleinen Strukturen die geometrische Aufsetzgenauigkeit (*alignment*) gewährleistet sein, die durch eine exakte Positionierbarkeit und einen relativen Positionsabgleich charakterisiert ist. Vorzugsweise, aber nicht einschränkend, wird als Material für die Stempel-Herstellung ein Elastomer, z.B. Polydimethylsiloxan (PDMS), verwendet.

Wenn starke Basen oder halogenisierte Kohlenwasserstoffe wie z.B. chlorierte Lösungsmittel in der Reaktion eingesetzt werden, ist es möglich, härtere aber dafür chemisch inertere Materialien für die Matrizen-Herstellung zu verwenden. Um einen gleichmäßigen Kontakt zur Substrat-Oberfläche zu gewährleisen, kann in diesem Fall eine Hybridstruktur aus einem harten und chemisch resistenten Kern und weichen Kontaktflächen aus elastischem Material konstruiert werden. Als hartes Material kann z.B. Glas, Silizium, Gold, Silber, Nickel oder andere Metalle sowie verschiedene Kunststoffe verwendet werden. Die Kontaktflächen der topologischen Strukturen können aus einem Elastomer wie PDMS oder anderen Siloxanen, Silikonen, gummiartigen Polymeren, Polyurethanen und weiteren formbaren elastischen Thermoplasten bestehen. Zusätzlich können chemische Modifikationen die chemische Resistenz des Stempels erhöhen. Diese können z.B. eine Erhöhung des Quervernetzungsgrads des Polymers, eine Verglasung der äußeren Schicht des Stempels, eine Oberflächenveredelung durch Aufbringen einer dünnen Schutzschicht aus einem Schutzpolymer oder einem Metall, oder sonstige geeignete chemische Modifikationen sein.

Zur Herstellung von strukturierten Stempeln werden Masterstrukturen z.B. in Form einer Siliziumscheibe (*silicon wafer*) oder als strukturiertes Glas mittels klassischer Photolithographie definiert und als Abgußform für das flüssige Prepolymer verwendet. Nach dem Aushärten läßt sich das elastische Polymer in der gewünschten dreidimensionalen Form abziehen. Zur Herstellung von Strukturen aus PDMS wird auf folgenden Übersichtsartikel verwiesen: Xia and Whitesides, Angew. Chem. Int. Ed. 37: 550-575 (1998).

PDMS ist für die Wellenlängen bis in den tieferen UV-Bereich transparent. Dies kann erfindungsgemäß dazu genutzt werden, um beispielsweise eine optische Kontrolle der Stempel-Positionierung relativ zum Substrat *(alignment)* zu gewährleisten. Ferner können bestimmte lichtsensitive Reaktionen *(light-sensitive reactions)* z.B. zur Ankopplung von Molekülen an die Substrat-Oberfläche durch Licht gesteuert werden.

Der Matrizen-Stempel kann erfindungsgemäß flach, walzenförmig, gewölbt oder konvex sein, oder eine sonstige für Kontaktdruckverfahren geeignete Form aufweisen.

Als Matrizen-Moleküle können erfindungsgemäß Oligomere und Polymere der Nukleotidklasse (DNA, RNA, PNA sowie ihre Derivate) dienen. Als Abbild-Moleküle können erfindungsgemäß nach dem beschriebenen Verfahren DNA, RNA oder Aptamere sowie deren insbesondere nukleaseresistente Derivate wie PNA oder thioRNA, sowie Proteine und Peptide in einer Polymerisations-Reaktion an der Matrize synthetisiert werden. Die mit dieser Methode synthetisierten Aptamere, die als Antikörper-analoge Bindemoleküle fungieren, können in verschiedenen Bindungs-Assays verwendet werden und in einigen Fällen die in Produktion teuren und aufwendigen Antikörper-Arrays funktionell ersetzen. Bei der Synthese von RNA können auch mit Aminosäure-Resten oder anderen funktionellen Gruppen modifizierte Nukleotide eingesetzt werden. Möglich wäre es auch Kohlenhydrat-Oligomere oder andere kombinatorisch synthetisierte Verbindungen an einer Matrize auf dem Stempel zu synthetisieren. Als Original-Array zum Kopieren vieler Abbild-Arrays können erfindungsgemäß alle Molekül-Arrays der Nukleotidklasse eingesetzt werden.

Die Erfindung ist nicht auf Polymerisations-Reaktionen beschränkt. Auch der Einsatz prozessierender Enzyme wie Proteasen, Nukleasen, Kinasen, Transaminasen, Methylasen, Synthetasen und anderer Enzyme bei der Herstellung von Abbild-Arrays nach dem beschriebenen Verfahren ist möglich. So kann ein Molekül auf dem Substrat durch einen oder mehrere gesonderte Stempelvorgänge prozessiert werden, wobei insbesondere eine Proteolyse, Phosphorylierung, Alkylierung, Glykosylierung, Methylierung oder weitere chemische Modifikationen eingeführt werden können.

Mit dem vorliegenden Verfahren lassen sich nicht nur Arrays mit punktartig in Form von Spots angeordneten Molekül-Gruppen herstellen. Moleküle, deren Gesamtheit eine Punktmatrix, eine kreisförmige, spiralförmige, streifenförmige, lineare oder sonstige geometrische Anordnung bildet, können ebenso an einer Matrize kopiert und ortstreu auf ein Substrat übertragen werden und sind damit Bestandteil der 5 Erfindung.

Die in der Erfindung beschriebenen Matrizen-Stempel können nicht nur als Vorlage zur Synthese von Molekülen verwendet werden. In einer weiteren Anwendung können mit Hilfe von Matrizen-Stempeln Moleküle aus bereits fertigen komplexen Gemischen isoliert und in einem vordefinierten Muster entsprechend der Lokalisation von Matrizen-Molekülen angeordnet werden. Auf diese Weise können z.B. Antikörper Arrays mit einem Matrizen-Stempel, auf dem Antigene angebracht sind, aus einem Antikörper-Gemisch wie Blutserum sehr schnell und einfach hergestellt werden. Umgekehrt lassen sich Proteine mit einer Antikörper-Matrize auf einem Array anordnen.

Ferner umfaßt die Erfindung die Verwendung derart hergestellter Molekül-Arrays für analytische Zwecke. So können die nach dem beschriebenen Verfahren hergestellten Arrays vorteilhaft für eine Vielzahl von Untersuchungen in der medizinischen und veterinärmedizinischen Diagnostik, der Artzneimittel-Entwicklung, der Qualitätskontrolle biologischer Agenzien, der Forensik, der Untersuchung von Pflanzenmetaboliten, der Analytik im Rahmen des Umweltschutzes, oder in der Forschung und Entwicklung eingesetzt werden.

Weitere Vorteile, Merkmale und Anwendungsmöglichkeiten der Erfindung werden nachstehend anhand der Ausführungsbeispiele mit Bezug auf die Zeichnungen beschrieben. In den Zeichnungen zeigen:
- Fig. 1: das grundlegende Prinzip des erfindungsgemäßen Verfahrens zur Herstellung von Arrays,
- Fig. 2: ein Ausführungsbeispiel des Verfahrens für die Herstellung eines DNA-Arrays,
- Fig. 3: eine Variante für die Herstellung eines DNA-Arrays ausgehend von einem anderweitig hergestellten Original-DNA-Array,
- Fig. 4: eine Variante für die Herstellung eines DNA-Arrays ausgehend von einem anderweitig hergestellten Original-DNA-Array, bei dem die Sequenzen unbekannt und die Moleküle mit ihren 3'-Enden an der Array-Oberfläche befestigt sind.
- Fig. 5: ein Ausführungsbeispiel des erfindungsgemäßen Verfahrens für die Herstellung eines Protein-Arrays,
- Fig. 6: eine Variante für die Herstellung eines Protein-Arrays ausgehend von einem anderweitig hergestellten Original-DNA-Array,
- Fig. 7: eine Variante des Verfahrens, bei der durch die Verwendung topologischer Stempel und das wiederholte lateral versetzte Kontaktdrucken kleinere Spot-Größen und eine höhere Dichte erreicht werden können als bei der Tropfenapplikation.

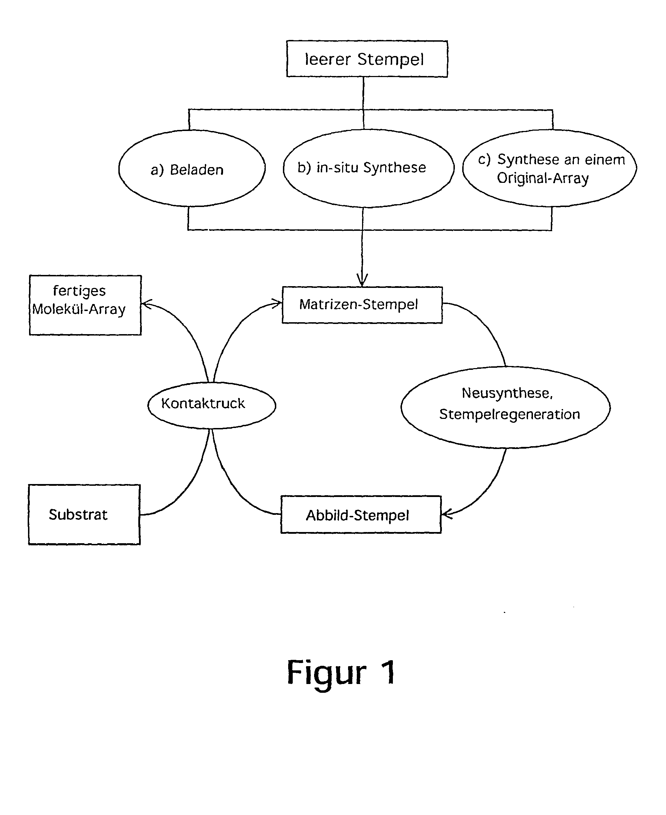

**Figur 1** zeigt das grundlegende Prinzip des in der Erfindung beschriebenen Verfahrens. Als erstes wird ein noch leerer Stempel mit den Matrizen-Molekülen beschichtet. Dies kann nach einer der drei folgenden Methoden erfolgen: a) durch das Beladen der Oberfläche mit fertigen Makromolekülen durch eine Tropfenapplikation z.B. mittels Drucknadeln oder Tintenstrahldrucker nach Stand der Technik; b) durch eine sequentielle *in situ*-Neusynthese der Makromoleküle aus einzelnen Komponenten auf der Stempel-Oberfläche nach Stand der Technik; c) durch eine Synthese an den Makromolekülen eines anderweitig synthetisierten Original-Arrays, die als Vorlage für diese Synthese dienen, nach dem erfindungsgemäßen Verfahren. In diesem Schritt wird die Form und der Inhalt des herzustellenden Arrays durch die Gestaltung des Matrizen-Stempels festgelegt.

lm zweiten Schritt werden die zu stempelnden Abbild-Moleküle durch eine Neusynthese an den Matrizen-Molekülen des Stempels hergestellt. Der Stempel wird im dritten Schritt mit einem Substrat in Kontakt gebracht, wodurch die Abbild-Moleküle auf die Substrat-Oberfläche übertragen werden. Es resultiert ein fertiges Molekül-Array mit einer definierten räumlichen Anordnung von Spots, die ein Spiegelbild des Marizen-Stempels bzw. eine Kopie des Original-Arrays darstellt. Der Matrizen-Stempel kann nach einer Regeneration (Neusynthese von Abbild- Molekülen) für eine neue Array-Herstellung verwendet werden. Der zweite und der dritte Schritt können nun mehrfach wiederholt werden, so daß eine Vielzahl von Molekül-Arrays in kurzer Zeit hergestellt werden kann.

### Beispiele

### a) Herstellung von DNA-Arrays

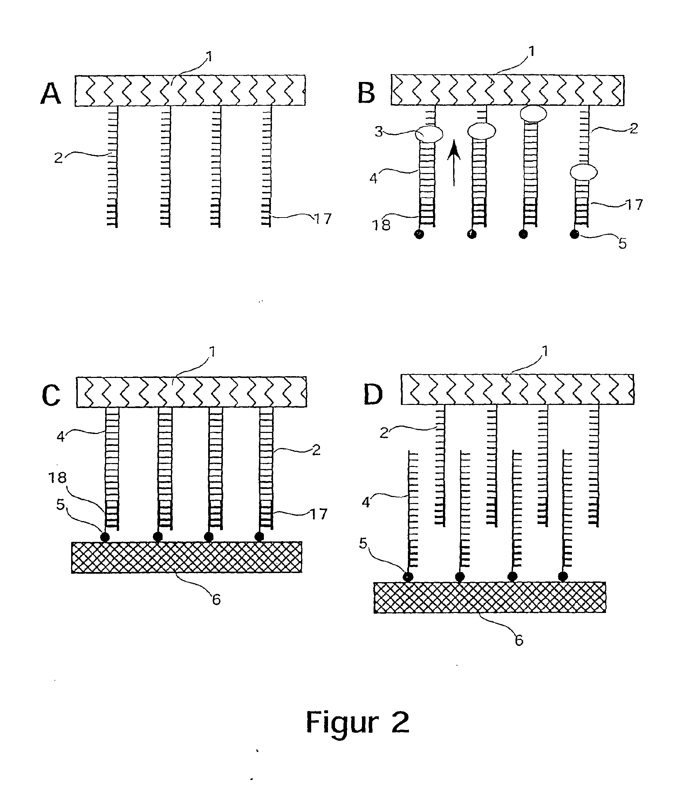

In Figur 2 ist vereinfacht die erste bevorzugte Ausführung der Erfindung dargestellt, bei der sowohl die Matrizen- als auch die Abbild-Moleküle DNA-Sequenzen sind. In diesem Beispiel werden die Array-Moleküle durch eine *in situ*-DNA-Polymerisation auf einer Substrat-Oberfläche hergestellt. Ein Standardverfahren kann verwendet werden, um die Matrizen-DNA-Moleküle **2** auf den leeren Matrizen-Stempel **1** aufzubringen **(Fig. 2A).** Die in Länge und Sequenz verschiedenen DNA-Einzelstränge sind den Zielsequenzen komplementär und stellen die Matrize für ihre Synthese dar. In einer bevorzugten Variante wird die Matrizen-DNA am 5'-Ende kovalent oder nicht-kovalent z.B. mittels eines Ankers auf der Stempel-Oberfläche befestigt und umfaßt beispielhaft und nicht ausschließlich sowie nicht notwendigerweise in der angegebenen Reihenfolge folgende Elemente:
- eine Verankerungsgruppe (z.B. ein Oligonukleotid einer definierten Sequenz, die komplementär zur Sequenz eines Oligonukleotides auf dem Stempel ist), ein Spacer, der die DNA mit dem Anker verbindet,
- die der Ziel-DNA komplementäre Sequenz,
- eine Primer-Bindungssequenz.

Die Primer-Bindungssequenz **17,** die bevorzugt aus acht bis zwanzig Nukleotiden besteht, ist identisch bei allen Matrizen-Molekülen auf dem Stempel **1**. Somit läßt sich durch den Einsatz eines der Primer-Bindungssequenz komplementären Universalprimers **18** die DNA-Sequenzen auf dem gesamten Array in einer parallelen Reaktion replizieren. Dazu wird der mit der Matrizen-DNA beladene Stempel in einem dem Fachmann bekannten Polymerisations-Cocktail, u.a. bestehend aus DNAPolymerase, Nukleotiden und Primern, inkubiert. An der Matrizen-DNA 2 auf dem Stempel **1** wird nun die Abbild-DNA 4 in einer Primer-Extinktions-Reaktion von einer DNA-Polymerase **3** synthetisiert (**Fig. 2B,** Pfeil), wobei die Polymerisation gleichzeitig an allen Spots geschieht. Die frisch synthetisierte Abbild-DNA bleibt vorerst am Ort der Synthese mit der DNA-Matrize hybridisiert. Beim nachfolgenden Kontaktdrucken werden alle Abbild-DNA-Moleküle **4** gleichzeitig und parallel auf die Ziel-Oberfläche **6** übertragen **(Fig. 2C).** Die Bindung der DNA an die Substrat-Oberfläche kann z.B. über einen Anker **5** erfolgen. Eine Destabilisierung der Wasserstoff-Brücken zwischen den beiden DNA-Strängen kann z.B. durch die Verwendung von Detergenzien oder Salzen, durch die Änderung des pH-Wertes, oder durch die Erhöhung der Temperatur erreicht werden. Bei der Trennung des Stempels **1** von der Substrat-Oberfläche **6** bleiben die Matrizen-Moleküle **2** auf dem Stempel zurück **(Fig. 2D),** die in den nächsten Stempel-Zyklen für weitere Polymerisations-Reaktionen als Vorlage verwendet werden können.

### b) Kopieren anderweitig hergestellter DNA-Arrays

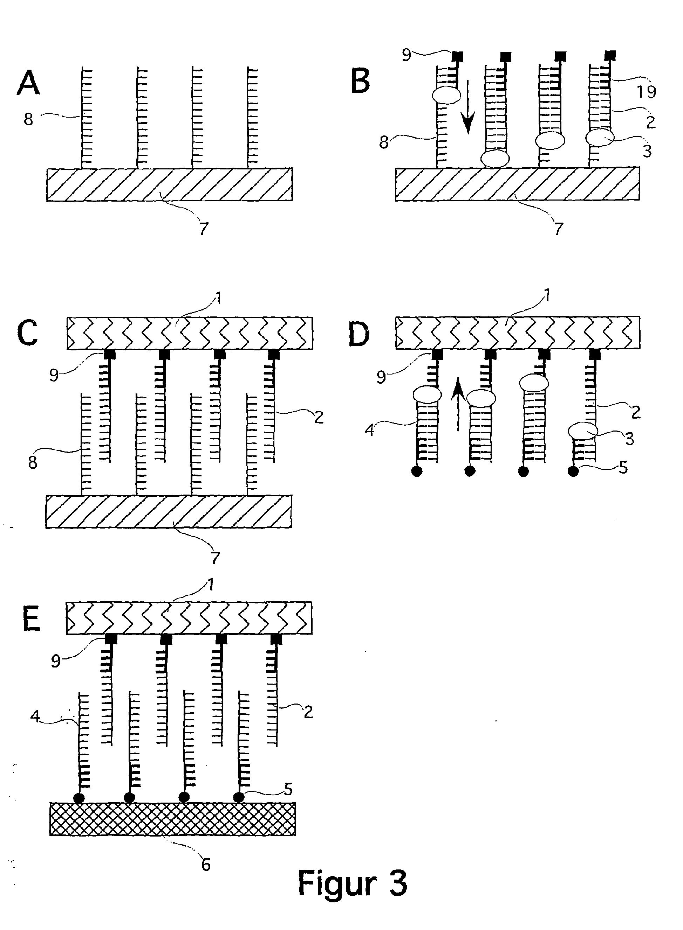

**Figur 3** zeigt vereinfacht ein Verfahren zur Vervielfältigung bereits existierender DNA-Arrays. Dazu wird zunächst ein anderweitig hergestelltes Original-Array 7 mit der darauf plazierten Original-DNA **8 (Fig. 3A)** als Vorlage zur Herstellung eines regenerierbaren DNA-Matrizen-Stempels verwendet. Wenn die DNA auf dem Original-Array nicht mit ihrem 3'-Ende an der Array-Oberfläche gebunden ist (das ist z.B. bei DNA-Arrays der Fall, die durch eine Tropfenapplikation hergestellt werden), und ihre Sequenz bekannt ist, werden für die Polymerisations-Reaktion unterschiedliche Primer **19** konstruiert, welche den Original-DNA-Sequenzen an ihren 3'-Enden komplementär sind. Die Synthese der Matrizen-DNA **2** erfolgt an der Original-DNA **8** in einer Primer-Extensions-Reaktion auf der Oberfläche des Original-Arrays **7** durch eine DNA-Polymerase **3 (Fig. 3B,** Pfeil). Das frisch synthetisierte Array von Matrizen-Molekülen **2** wird anschließend auf den noch leeren Stempel **1** in einem parallelen Schritt übertragen und z.B. mit Hilfe von Ankern **9** gebunden **(Fig. 3C).** Die auf dem Stempel immobilisierte Matrizen-DNA kann nun wie oben beschrieben als Vorlage für die wiederholte Synthese von Abbild-Arrays benutzt werden **(Fig. 3D** und **3E).**

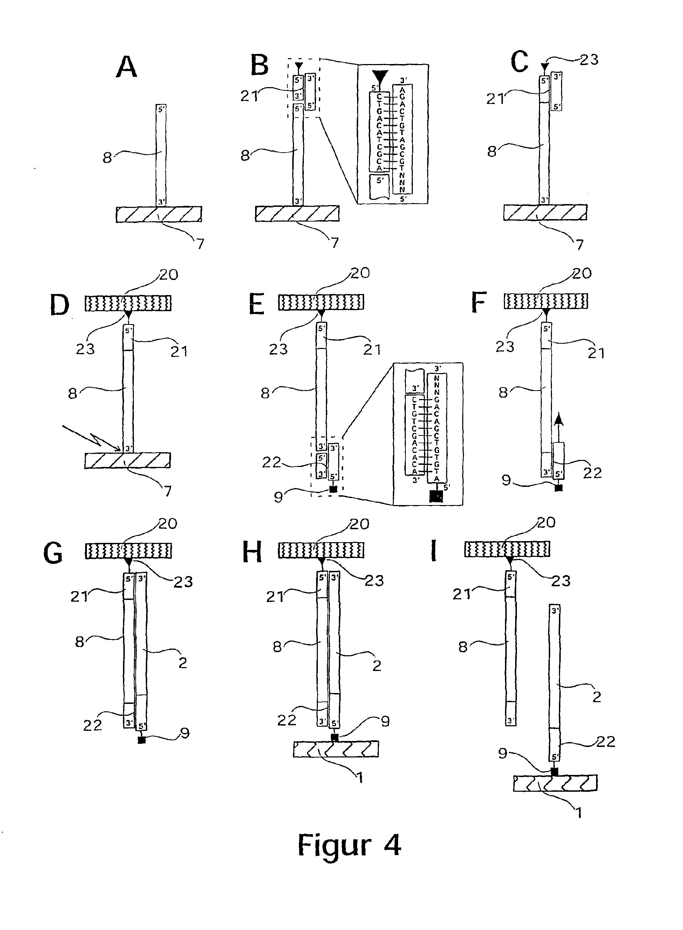

Müssen Original-Arrays vervielfältigt werden, bei denen unbekannte DNASequenzen mit ihren 3'-Enden an der Oberfläche befestigt sind, so können speziell konstruierte Adaptor-Elemente anstelle von Primern zur Polymerisation verwendet werden. Figur **4** zeigt eine bevorzugte Ausführungsvariante zur Vervielfältigung eines DNA-Arrays, bei dem die Sequenz unbekannt ist und die DNA-Moleküle **8** mit ihren 3'-Enden an der Array-Oberfläche **7** befestigt sind **(Fig. 4A).** So sind z.B. Arrays aufgebaut, die mittels klassischer Festphasensynthese z.B. in einem Phosphoamidit-Verfahren oder in einem lithographischen Aufbringungsverfahren hergestellt werden. In diesem Fall muß zuerst die Orientierung der Original-DNA an der Oberfläche geändert werden, um ihr 3'-Ende für eine Polymerase zugänglich zu machen. Dies kann z.B. durch die Verwendung von Adaptor-Elementen, Spaltung an der DNA-Bindungsstelle und Übertragung auf einen Hilfsstempel erreicht werden. Das erste Adaptor-Element **21** kann beispielhaft und nicht ausschließlich folgende Sequenz haben:
5'-CTG-ACA-TCG-CA-3'
3'-A-GAC-TGT-AGC-GTN-N(N)-5',
wobei N für eines der Nukleotide A, T, G oder C steht. Diese zwei bis drei permutierten ("wobble") Nukleotide sollen die Hybridisierung an die DNA mit unbekannter Sequenz an ihrem 5'-Ende ermöglichen **(Fig. 4B).** Nach der Ligation des ersten Adaptors **21** an die Original-DNA 8 **(Fig. 4C)** z.B. mit Hilfe einer T4-Ligase wird die Original-DNA in einem Kontaktdruck-Verfahren durch eine parallele und ortstreue Aufnahme auf den leeren Hilfsstempel **20** übertragen **(Fig. 4D).** Dabei kann es nötig sein, die DNA durch ein enzymatisches Schneiden z.B. mit einer Restriktionsendonuklease oder eine chemische Spaltung z.B. mit einer KOH-Lösung in Isopropanol von der Array-Oberfläche **7** zu trennen **(Fig. 4D,** Blitz). Die Bindung der Original-DNA **8** an die Oberfläche des Hilfsstempels **20** kann durch einen Anker **23,** z.B. ein Biotin, der am ersten Adaptor-Element angebracht ist, ermöglicht werden. In diesem Schritt wird die DNA an ihrem 5'-Ende an der Oberfläche befestigt, so dass sie für eine Polymerisation eine günstige Orientierung bekommt. Im nächsten Schritt wird ein zweites, nach dem gleichen Prinzip konstruiertes aber nicht notwendigerweise die gleiche Sequenz aufweisendes Adaptor-Element **22** beispielhaft und nicht ausschließlich mit einer Sequenz
5'-CTG-TCG-ACA-CA-3'
3'-NNN-GAC-AGC-TGT-GTA-5'
am 3'-Ende der Original-DNA **8** hybridisiert **(Fig. 4E)** und ligiert **(Fig. 4F).** Das zweite Adaptor-Element enthält einen Anker **9** zur Bindung der DNA an die Stempel-Oberfläche und eine Primer-Bindungssequenz zur Hybridisierung eines Universalprimers für die nachfolgende Synthese der Abbild-DNA. Im Polymerisations-Cocktail, enthaltend DNA-Polymerase und Nukleotide, wird nun die Matrizen-DNA 2 ausgehend von der Sequenz des zweiten Adaptors **22** in einer Primer-Extensions-Reaktion an der Original-DNA **8** synthetisiert **(Fig. 4F,** Pfeil, und **4G).** Die frisch synthetisierte Matrizen-DNA **2** wird an die Oberfläche des Matrizen-Stempels **1** durch einen Kontaktdruck übertragen und z.B. über einen Anker **9** gebunden **(Fig. 41).** An der Matrizen-DNA auf dem Matrizen-Stempel kann nun die Abbild-DNA in einer Polymerisations-Reaktion wie im Beispiel **a)** beschrieben synthetisiert und an die Ziel-Oberfläche mittels eines Kontaktdrucks übertragen werden. In diesem und weiteren Stempel-Zyklen wird bei der Polymerisations-Reaktion ein an alle DNA-Moleküle bindender Universalprimer eingesetzt, welcher der Primer-Bindungssequenz des ersten Adaptors komplementär ist. In diesem Fall hat der Universalprimer die Sequenz
5'-CTG-ACA-TCG-CA-3'

Die Anwendung des Verfahrens zum Kopieren von DNA-Array mit bekannten Sequenzen, die mit ihren 3'-Enden an der Oberfläche gekoppelt sind, bzw. von Arraysmit unbekannten Sequenzen, deren 3'-Enden frei sind, stellen Teilbeispiele der beiden oben aufgeführten Fällen dar. Deren Ausführung ergibt sich aus den Beispielen **a)** und **b)** und kann vom Fachmann vorgenommen werden.

### c) Herstellung von Protein-Arrays

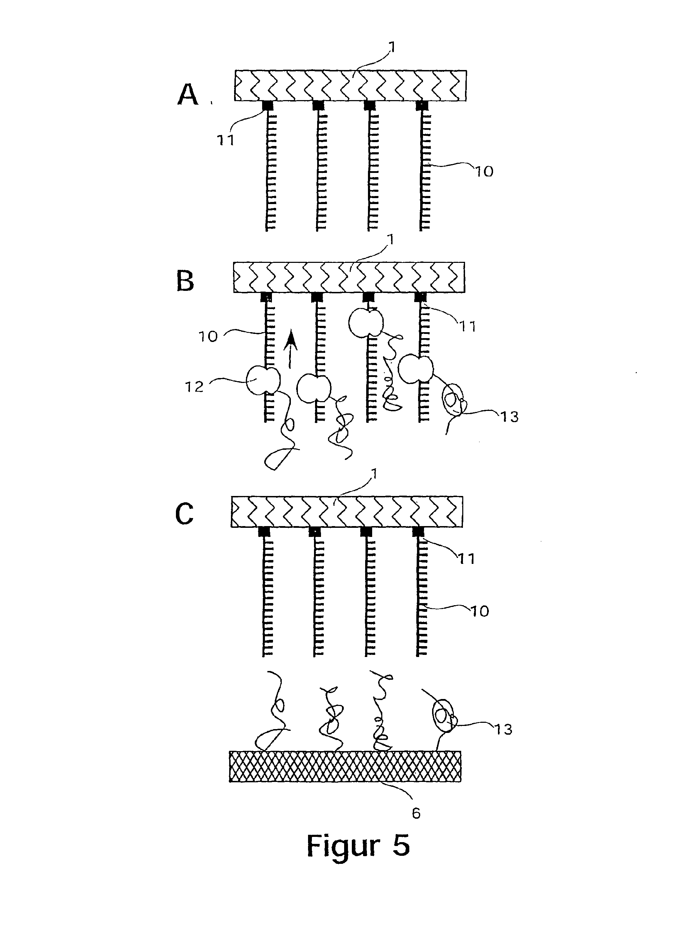

Bei dem in **Figur 5** dargestellten Ausführungsbeispiel handelt es sich um eine weitere bevorzugte Anwendung des beschriebenen Verfahrens für die Herstellung von Protein-Arrays. Dabei werden Molekül-Bibliotheken aus Polypeptiden auf der Substrat-Oberfläche durch eine *in situ*-Translation hergestellt. Die Positionsinformation ist für jedes einzelne Protein-Spot durch die Anordnung der dafür kodierenden RNA auf der Stempel-Oberfläche festgelegt.

Die RNA-Moleküle **10** werden zunächst in einem Standardverfahren z.B. durch eine Tropfenapplikation auf die Stempel-Oberfläche **1** aufgetragen und kovalent oder nichtkovalent z.B. über einen Anker **11** gebunden **(Fig. 5A).** Die gebundenen RNAMoleküle dienen nun als Matrize für die Herstellung von Proteinen. Um die Stabilität der RNA-Moleküle zu erhöhen, können chemisch modifizierte Nuklease-resistente RNA-Moleküle eingesetzt werden. Nach Stand der Technik können dies insbesondere thio-RNA sein. Alle Formen translatierbarer Polynukleotide werden im Folgenden zur Vereinfachung als RNA bezeichnet. Solche Moleküle können z.B. nach Stand der Technik synthetisch hergestellt werden.

Die Sequenz der als Matrize verwendeten RNA-Moleküle umfaßt beispielhaft und nicht ausschließlich sowie nicht notwendigerweise in der angegebenen Reihenfolge folgende Elemente:
- eine Verankerungsgruppe (z.B. ein Oligonukleotid einer definierten Sequenz, die komplementär zur Sequenz eines Oligonukleotides auf dem Stempel ist),
- eine Abfolge der genetischen Elemente, die zur Initiation und Fortführung einer enzymatischen Translations-Reaktion benötigt werden, im Besonderen eine Ribosomen-Bindungsstelle und ein Start-Codon, z.B. AUG,
- eine Sequenz, die ein Oligopeptid ("Tag") kodiert, das eine hohe Affinität an ein Bindemolekül auf der Substrat-Oberfläche besitzt,
- die Sequenz des zu druckenden Proteins,
- eine Sequenz, die ein Oligopeptid-Spacer kodiert, der bewirkt, daß das Protein in seiner ganzen Länge das Ribosom verlassen kann, ohne daß der Komplex mit der RNA vorzeitig zerfällt, z.B. eine oder mehrere Wiederholungen einer Sequenz, die das Oligopeptid (Gly)₄Ser kodiert,
- zur Verhinderung der Auflösung von Transkriptionskomplexen endet die RNA in bevorzugter Ausführung nicht mit einem Stop-Codon; alternativ können besondere Signale oder chemische Modifizierung auf der RNA verwendet werden.

Der mit der RNA beladene Stempel wird in einem *in vitro*-Translations-Cocktail inkubiert, das die zum Ablauf einer Translations-Reaktion notwendigen Komponenten enthält. Solche Suspensionen können z.B. aus *E. coli* oder Kaninchen-Retikulozyten hergestellt werden und sind dem Fachmann bekannt.

Wenn lange oder komplexe Polypeptid-Sequenzen hergestellt werden müssen, können zusätzliche Komponenten, insbesondere Chaperone, Chaperonine oder andere die Faltung unterstützende Substanzen, wie z.B. Glutathion, dem *in vitro*-Translations-Cocktail zugefügt werden. Dies ist insbesondere empfehlenswert, wenn die Polypeptid-Sequenzen Fragmente komplexer Proteine sind, die natürlicherweise nicht im Zytoplasma einer Zelle gefaltet werden. Typische Beispiele dafür sind Mitglieder der Immunoglobulin-Superfamilie wie Zelloberflächen-Rezeptoren, Antikörper oder T-Zell-Rezeptoren.

Die Synthese der Polypeptid-Sequenzen **13** an der RNA durch das Ribosom **12** endet, wenn das Ende der RNA erreicht ist. Der Translationskomplex zerfällt allerdings nicht, da in einer bevorzugeten Ausführungsvariante die RNA kein Stop-Codon enthält. Der Stempel **1,** der jetzt zusätzlich zu der Matrizen-RNA **10** noch die neu synthetisierten Polypeptide **13** enthält, welche am Ort ihrer Synthese immobilisiert 5 sind **(Fig. 5B),** wird aus dem *in vitro*-Translations-Cocktail entnommen und mit physiologischem Puffer gewaschen.

Der Stempel 1 mit den neusynthetisierten Proteinen **13** wird nun auf die zu bestempelnde Oberfläche **6** aufgedrückt. Erfindungsgemäß kann ein Hilfsstoff eingesetzt werden, welcher Ribosomen oder Translationskomplexe destabilisiert, typischerweise ein Chelatbildner wie EDTA oder EGTA. Dabei löst sich der Komplex aus RNA **10** und Protein **13,** und Proteine werden an die zu bestempelnde Oberfläche **6** übertragen **(Fig. 5C).** Nach Entfernung des Stempels weist die bestempelte Oberfläche eine Beschichtung mit Proteinen auf, die spiegelbildlich zur Anordnung der kodierenden RNA auf dem Substrat ist. Somit ist gewährleistet, daß ein an einer bestimmten Stelle dieses Protein-Arrays durch Bindung eines Analyts generiertes Signal eindeutig einem Protein mit bestimmter Sequenz zugeordnet werden kann. Nach der oben beschriebenen Methode können vorteilhaft Arrays mit Peptiden, Proteinen, Antikörpern oder Antikörperfragmenten, insbesondere scFv-Fragmenten hergestellt werden.

### d) Translation anderweitig hergestellter DNA-Arrays

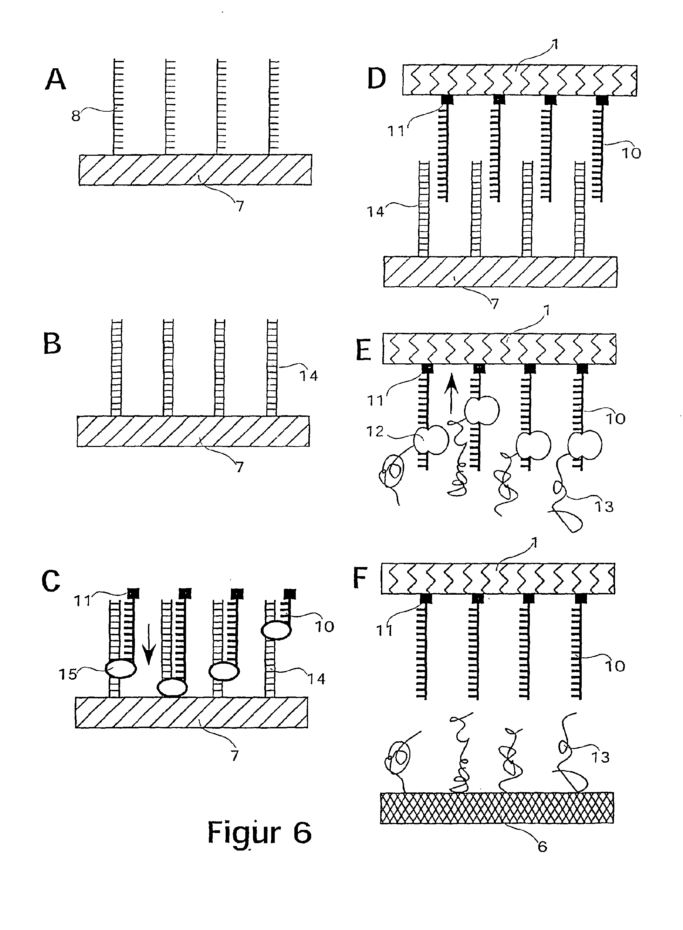

Figur 6 zeigt eine Erweiterung des oben beschriebenen Beispiels auf die Herstellung eines Protein-Arrays ausgehend von einem anderweitig hergestellten Original-Array **7,** das die einzelsträngige **DNA 8** auf der Oberfläche trägt **(Fig. 6A).** In diesem Fall wird zunächst die doppelsträngige DNA **14** in einer Polymerisations-Reaktion mit Hilfe einer DNA-Polymerase nach Stand der Technik hergestellt **(Fig. 6B).** Wenn die Original-DNA nicht mit ihrem 3'-Ende an der Array-Oberfläche befestigt ist, kann der komplementäre Strang z.B. in einer Primer-Extensions-Reaktion unter Einsatz eines speziell konstruierten Primers synthetisiert werden. Der Primer kann eine Sequenz mit den für die Transkription und Translation nötigen Signalen enthalten. Zur Transkription kann z.B. die Sequenz des T7-Promotors verwendet werden. Die Primer-Bindung an die DNA-Stränge kann entweder durch die Verwendung von komplemänteren Sequenzen, wenn die Original-Sequenzen bekannt sind, oder durch die Einführung von permutierten ("wobbled") Nukleotiden, wie im Beispiel **b)** beschrieben, ermöglicht werden. Alternativ kann die Sequenz mit den für die Transkription und Translation nötigen Signalen durch eine enzymatische oder chemische Synthese nach Stand der Technik direkt an den DNA-Original-Einzelstrang an seinem 3'-Ende angebracht werden. Wenn Arrays mit den an ihren 3'-Enden an der Array-Oberfläche befestigten DNA-Molekülen translatiert werden sollen, müssen diese zuerst in die umgekehrte Orientierung gebracht werden. Dies kann z.B. über einen Hilfsstempel mittels speziell konstruierter Adaptor-Elemente wie im Beispiel **b)** beschrieben geschehen.

Im nächsten Schritt wird an der doppelsträngigen DNA-Matritze **14** die aufzubringende RNA **10** in einer Transkriptions-Reaktion von einer RNA-Polymerase synthetisiert **(Fig. 6C).** Alternativ kann auf die Herstellung der Doppelstrang-DNA und anschließende Transkription verzichtet werden, indem kleine RNA-Fragmente mit zufälligen *(random)* Sequenzen an die einzelsträngige Original-DNA hybridisiert und in einem Cocktail enthaltend einzelne Nukleotide durch eine T4-RNA-Ligase zueinem der Original-DNA komplementären RNA-Srang zusammenligiert werden. Die T4-RNA-Ligase kann ebenfalls dazu verwendet werden, um einen an ein Nukleotid oder ein Oligonukleotid gekoppelten Anker an das 5'-Ende der frisch synthetisierten RNA anzubringen.

Die frisch synthetisierte RNA **10** wird anschließend auf einen Stempel **1** übertragen und kovalent oder nicht-kovalent z.B. über einen Anker **11** an die Stempel-Oberfläche gebunden **(Fig. 6D).** Der auf diese Weise hergestellte Stempel wird weiter für die wiederholbare Synthese und Übertragung von Proteinen auf eine Array-Oberfläche wie oben im Beispiel **c)** beschrieben eingesetzt **(Fig. 6E** und **6F).** Mit diesem Verfahren ist also Umkopieren Transkription und Translation von DNA-Arrays zur Herstellung von Protein-Arrays möglich. Die beschriebene Anwendung könnte z.B. vorteilhaft bei EST Microarrays (expressed sequence tags) für die Analyse von Sequenzen mit unbekannten Funktionen eingesetzt werden.

Die beschriebene erfindungsgemäße ortsgebundene Vervielfältigung auch hochkomplexer Protein-Bibliotheken erlaubt eine wesentlich preiswertere Herstellung von Protein-Arrays als mit bisherigen Methoden. Die Anwendung des Verfahrens für die Herstellung von Protein-Arrays eignet sich auch zur Verbesserung der Qualität und Vereinfachung der Herstellung von Antikörper-Arrays oder Arrays funktionsäquivalenter Moleküle, wie Antikaline, Fibronektin-Varianten, Einzeldomän-Antikörper, Affibodies oder auf anderen Backbones basierende Bindemoleküle. Die Antikörper bzw. funktionsäquivalente Moleküle müssen vor dem Auftragen auf das Substrat nicht einzeln hergestellt und gereinigt werden, sondern können in einer gemeinsamen Reaktion gleichzeitig und hochparallel synthetisiert werden. lm erfindungsgemäßen Verfahren muß lediglich die wesentlich einfacher zu reinigende und in ihrer Qualität zu kontrollierende DNA einzeln hergestellt und gereinigt werden.

Die Erfindung umfaßt ferner die Verwendung derart hergestellter Protein-Arrays für analytische Zwecke. In einer beispielhaften Anwendung können Proteine verschiedener infektiöser Pathogene oder auch Antikörper gegen Pathogene auf ein Array aufgebracht werden. Patienten mit unspezifischem Fieber können dann anhand einer einzigen Blut- oder Gewebeprobe mit einem solchen Protein-Array gleichzeitig auf eine Vielzahl unterschiedlicher Krankheitserreger untersucht werden.

### e) Verringerung der Spot-Größe und Erhöhung der Spot-Dichte

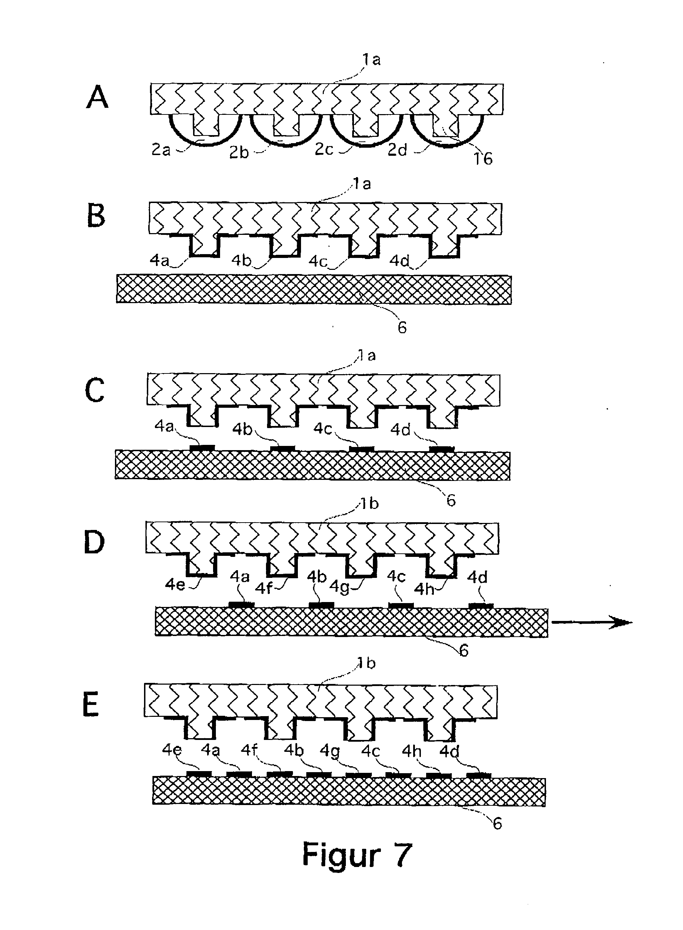

**Figur 7** zeigt vereinfacht ein Anwendungsbeispiel der beschriebenen Erfindung für die Herstellung von Molekül-Arrays mit einer kleineren Spot-Größe und einer höheren Spot-Dichte als durch eine Tropfenapplikation hergestellte Arrays. Angenommen, durch eine Tropfenapplikation können Arrays mit einem Spot- Durchmesser von 40 µm und einem Abstand zwischen den benachbarten Spots von 100 µm generiert werden. Mit dem erfindungsgemäßen Verfahren wird in diesem Beispiel ein Molekül-Array mit einem Spot-Durchmesser von 10 µm und einem Abstand von 50 µm zwischen den Spots hergestellt. Dazu wird im ersten Schritt ein topologischer Stempel **1a** generiert, auf dem im Querschnitt runde oder andersförmige Erhebungen **16** mit einem Durchmesser von 10 µm in einem Abstand von 100 µm lokalisiert sind. Mittels einer Tropfenapplikation werden Lösungen unterschiedlicher Matrizen-Moleküle **2a** bis **2d** auf die Erhebungen des Stempels in einem Raster von 100 µm aufgetragen **(Fig. 7A).** Nach dem Aufbringen der Matrizen-Moleküle und dem Trocknen der Spots werden an diesem Matrizen-Array die entsprechenden Abbild-Moleküle **4a** bis **4d** nach dem oben beschriebenen Verfahren synthetisiert **(Fig. 7B).** Als nächstes wird der Stempel mit dem Substrat **6** in Kontakt gebracht **(Fig. 7C).** Bei diesem Vorgang werden nur die Moleküle auf die Substrat-Oberfläche übertragen, die auf der proximalen Fläche der Erhebungen lokalisiert sind, da nur diese Moleküle in einen direkten Kontakt mit der Substrat-Oberfläche kommen. Dadurch wird in diesem Schritt der Spot-Durchmesser von 40 µm auf 10 µm verringert. Beim nächsten Stempelvorgang verwendet man einen neuen Stempel **1b,** der in diesem Beispiel die gleiche Topologie aber unterschiedliche Molekül-Spots **4e** bis **4h** aufweist **(Fig. 7D).** Um eine höhere Spot-Dichte zu erreichen, wird das Substrat **6** dabei um 50 µm entlang seiner x-Achse verschoben **(Fig. 7D,** Pfeil). Bei einem Kontaktdruck kommen die neuen Spots **4e, 4f, 4g** und **4h** genau zwischen die zuvor aufgetragenen Spots **4a, 4b, 4c** und 4e in einem Abstand von 50 µm auf die Substrat-Oberfläche **6 (Fig. 7E).** Bei den nächsten beiden Stempelvorgängen werden **10** jeweils neue Stempel benutzt, und das Substrat wird um 50 µm entlang seiner y- bzw. x, y-Achse relativ zu seiner Ausgangsposition verschoben. Insgesamt werden vier Stempelvorgänge mit vier unterschiedlichen Stempeln betätigt. In dem gewählten Beispiel wird die Spot-Dichte vervierfacht und die Spot-Größe auf ein Viertel reduziert.

Die vorliegende Erfindung ist nicht auf die vorstehend beschriebenen Ausführungsbeispiele beschränkt. Vielmehr sind viele Abwandlungen möglich, die durch Fachleute erzielbar sind, und damit im Bereich dieser Erfindung eingeschlossen sind.

## Patentansprüche

1. Verfahren zur Herstellung von in einem definierten Muster angeordneten Molekül-Bibliotheken auf einem Substrat, **gekennzeichnet durch**
- die Herstellung mindestens eines Matrizen-Stempels, der eine definierte Anordnung von Matrizen-Molekülen beinhaltet,
- die ortstreue biochemische oder chemische *in situ*-Synthese von Abbild-Molekülen an den als Vorlage dienenden Matrizen-Molekülen auf der Oberfläche des Matrizen-Stempels,
- die Übertragung der synthetisierten Abbild-Moleküle auf ein Festkörper- oder Polymer-Substrat mittels Kontaktdruck unter Beibehaltung der räumlichen Anordnung der Abbild-Moleküle auf dem Substrat,
- die Wiederholbarkeit der Synthese- und Übertragungsschritte.

2. Verfahren nach Anspruch 1, **dadurch gekennzeichnet, daß** die Matrizen-Moleküle auf dem Matrizen-Stempel sowie die Abbild-Moleküle auf der Substrat-Oberfläche in Form einer Punktmatrix, einer kreisförmigen, spiralförmigen, streifenförmigen, linearen oder sonstigen geometrischen oder stochastischen Struktur angeordnet sind.

3. Verfahren nach einem der Ansprüche 1 oder 2, **dadurch gekennzeichnet, daß** die auf der Substrat-Oberfläche synthetisierten Molekül-Bibliotheken zur Durchführung von parallelen Bindungs-Reaktionen eingesetzt werden können.

4. Verfahren nach einem der Ansprüche 1 bis 3, **dadurch gekennzeichnet, daß** der Stempel mindestens zum Teil aus einem elastischen Material, vorzugsweise Polydimethylsiloxan besteht.

5. Verfahren nach einem der Ansprüche 1 bis 4, **dadurch gekennzeichnet, daß** zwei oder mehr unterschiedliche Stempel zur Herstellung eines Arrays benutzt werden.

6. Verfahren nach einem der Ansprüche 1 bis 5, **dadurch gekennzeichnet, daß** die Matrizen-Moleküle auf dem Stempel durch eine *in situ*-Synthese an den als Vorlage dienenden Molekülen eines anderweitig hergestellten Molekül-Arrays hergestellt werden.

7. Verfahren nach einem der Ansprüche 1 bis 5, **dadurch gekennzeichnet, daß** die Matrizen-Moleküle auf dem Stempel durch eine chemische oder eine durch elektrische Felder oder Licht gesteuerte in situ-Synthese hergestellt werden.

8. Verfahren nach einem der Ansprüche 1 bis 5, **dadurch gekennzeichnet, daß** das Aufbringen der Matrizen-Moleküle auf den Stempel durch eine Tropfenapplikation geschieht.

9. Verfahren nach einem der Ansprüche 1 bis 5, **dadurch gekennzeichnet, daß** der Stempel aus einem für eine bestimmte Wellenlänge transparenten Material hergestellt ist und so über die ortsselektive Lichtleitung die Durchführung photochemischer Reaktionen oder nahfeldoptischer Prozesse zur Bindung oder Synthese der Matrizen-Moteküle auf der Stempel-Oberfläche ermöglicht.

10. Verfahren nach einem der Ansprüche 1 bis 9, **dadurch gekennzeichnet, daß** der Stempel aus einem für eine bestimmte Wellenlänge transparenten Material hergestellt ist und so über die ortsselektive Lichtleitung eine Kontrolle der relativen Positionierung des Stempels auf der Subsstrat-Oberfläche ermöglicht.

11. Verfahren nach einem der Ansprüche 1 bis 10, **dadurch gekennzeichnet, daß** die Matrizen-Moleküle DNA, RNA oder ihre nukleaseresistenten Derivate wie PNA oder thioRNA sind.

12. Verfahren nach einem der Ansprüche 1 bis 10, **dadurch gekennzeichnet, daß** die Abbild-Moleküle DNA, RNA oder Aptamere, oder ihre insbesondere nukleaseresistenten Derivate wie PNA oder thioRNA sind.

13. Verfahren nach einem der Ansprüche 1 bis 10, **dadurch gekennzeichnet, daß** die Abbild-Moleküle Peptide, Proteine, Antikörper oder Antikörperfragmente, insbesondere scFv-Fragmente, oder dazu funktionsäquivalente Moleküle, insbesondere Anticaline, Fibronektin-Teildomänen oder einzelne Antikörper-Domänen sind.

14. Verfahren nach einem der Ansprüche 1 bis 10, **dadurch gekennzeichnet, daß** die Abbild-Moleküle Kohlenhydrate oder kombinatorisch synthetisierte Verbindungen sind.

15. Verfahren nach einem der Ansprüche 1 bis 14, **dadurch gekennzeichnet, daß** die Abbild-Moleküle nach der Synthese insbesondere durch Proteolyse, Phosphorylierung, Alkylierung. Glykosylierung, Methylierung oder chemische Behandlung modifiziert werden.

16. Verfahren nach Anspruch 15, **dadurch gekennzeichnet, daß** die Modifikation der Abbild-Moleküle in einem oder mehreren Stempelvorgängen erfolgt.

17. Verfahren nach einem der Ansprüche 1 bis 16, **dadurch gekennzeichnet, daß** die Abbild-Moleküle kovalent oder nicht-kovalent gebundene Anker oder chemische Gruppen enthalten, welche die Bindung an die zu bedruckende Substrat-Oberfläche vermitteln.

18. Verfahren nach Anspruch 17, **dadurch gekennzeichnet, daß** die zu bedruckende Substrat-Oberfläche das entsprechende molekulare Gegenstück zur Bindung des Ankers oder die mit der chemischen Gruppe des Ankers reagierende Substanz enthält.

19. Verfahren nach Anspruch 18, **dadurch gekennzeichnet, daß** der Anker und sein molekulares Gegenstück auf der zu bedruckenden Substrat-Oberfläche z.B. jeweils komplementäre Nukleotid-Sequenzen, oder ein Antikörper-Fragment und ein Antigen, oder ein S-Peptid und ein S-Protein, oder ein Biotin und ein Streptavidin, oder einen Liganden und einen Rezeptor, oder jeweils umgekehrt enthalten.

20. Verfahren nach einem der Ansprüche 1 bis19, **dadurch gekennzeichnet, daß** der Stempel topologische Strukturen in Form eines Reliefs auf der Oberfläche aufweist.

21. Verfahren nach Anspruch 20, **dadurch gekennzeichnet, daß** der strukturierte Stempel topologische Strukturen enthält, die kleinere Dimensionen als die auf dem Stempel durch ein Standardverfahren, insbesondere eine Tropfenapplikation, aufgetragenen Molekül-Spots aufweisen, bevorzugt kleiner als 10 µm, insbesondere kleiner als 2 µm.

22. Verfahren nach Anspruch 21, **dadurch gekennzeichnet, daß** auf dem Substratkleinere Molekül-Spots als die auf dem strukturierten Stempel durch ein Standardverfahren, insbesondere eine Tropfenapplikation, aufgetragenen Spots generiert werden, bevorzugt kleiner als 10 µm, insbesondere kleiner als 2 µm.

23. Verfahren nach einem der Ansprüche 21 oder 22, **dadurch gekennzeichnet, dass** durch das wiederholte versetzte Kontaktdrucken ein Array mit einer höheren Spot-Dichte als die auf dem strukturierten Stempel durch ein Standardverfahren, insbesondere eine Tropfenapplikation, generierte Spot-Dichte hergestellt wird.

24. Verfahren nach Anspruch 20, **dadurch gekennzeichnet, daß** der strukturierte Stempel topologische Strukturen enthält, die kleinere Dimensionen als die Molekül-Spots auf dem zur Vorlage für die Molekül-Synthese dienenden anderweitig hergestellten Molekül-Array aufweisen, bevorzugt kleiner als 10 µm, insbesondere kleiner als 2 µm.

25. Verfahren nach Anspruch 24, **dadurch gekennzeichnet, daß** auf dem Substrat kleinere Molekül-Spots als die Spots auf dem zur Vorlage für die Molekül-Synthese dienenden anderweitig hergestellten Molekül-Array generiert werden, bevorzugt kleiner als 10 µm, insbesondere kleiner als 2 µm.

26. Verfahren nach einem der Ansprüche 24 oder 25, **dadurch gekennzeichnet, daß** durch das wiederholte versetzte Kontaktdrucken ein Array mit einer höheren Dichte als auf dem zur Vorlage für die Molekül-Synthese dienenden anderweitig hergestellten Molekül-Array generiert wird.

27. Verfahren nach einem der Ansprüche 1 bis 26, **dadurch gekennzeichnet, daß** es zur Verbesserung der Qualität und zur Vereinfachung der Herstellung von Protein- und insbesondere von Antikörper-Arrays oder Arrays funktionsäquivalenter Moleküle, insbesondere Anticaline, Fibronektin-Teildomänen, Antikörper-Einzelketten oder Antikörper-Fragmente,eingesetzt werden kann.

28. Verfahren nach Anspruch 27, **dadurch gekennzeichnet, daß** die Protein- bzw. Antikörper-Arrays oder Arrays funktionsäquivalenter Moleküle bei der Diagnostik verschiedener Pathogene eingesetzt werden.

## Claims

1. Method of preparing molecule libraries arranged in a defined pattern on a substrate, **characterized by**
□ preparation of at least one template die which comprises a defined arrangement of template molecules,
□ true-to-location biochemical or chemical *in situ* synthesis of image molecules on the template molecules serving as models on the surface of the template die,
□ transfer of the synthesized image molecules to a solid or polymeric substrate by means of contact printing, with the spatial arrangement of the image molecules on the substrate being retained,
□ repeatability of the synthesis and transfer steps.

2. Method according to Claim 1, **characterized in that** the template molecules on the template die and the image molecules on the substrate surface are arranged in the form of a dot matrix, a circular, helical, strip-shaped, linear or other geometrical or stochastic structure.

3. Method according to either of Claims 1 and 2, **characterized in that** the molecule libraries synthesized on the substrate surface can be used for carrying out parallel binding reactions.

4. Method according to any of Claims 1 to 3, **characterized in that** the die comprises, at least partially, an elastic material, preferably polydimethylsiloxane.

5. Method according to any of Claims 1 to 4, **characterized in that** two or more different dies are utilized for preparing an array.

6. Method according to any of Claims 1 to 5, **characterized in that** the template molecules on the die are prepared by *in situ* synthesis on the molecules of a differently prepared molecule array, which serve as models.

7. Method according to any of Claims 1 to 5, **characterized in that** the template molecules on the die are prepared by a chemical or an electric field-or light-controlled *in situ* synthesis.

8. Method according to any of Claims 1 to 5, **characterized in that** the template molecules are applied to the die by way of application in drop form.

9. Method according to any of Claims 1 to 5, **characterized in that** the die is made of a material transparent for a particular wavelength and thus enables, via location-selective light conduction, photochemical reactions or near-field optical processes for binding or synthesizing the template molecules on the die surface to be carried out.

10. Method according to any of Claims 1 to 9, **characterized in that** the die is made of a material transparent for a particular wavelength and thus makes it possible to control relative positioning of the die on the substrate surface via location-selective light conduction.

11. Method according to any of Claims 1 to 10, **characterized in that** the template molecules are DNA, RNA or their nuclease-resistant derivatives such as PNA or thioRNA.

12. Method according to any of Claims 1 to 10, **characterized in that** the image molecules are DNA, RNA or aptamers or their, in particular nuclease-resistant, derivatives such as PNA or thioRNA.

13. Method according to any of Claims 1 to 10, **characterized in that** the image molecules are peptides, proteins, antibodies or antibody fragments, in particular scFv fragments, or molecules functionally equivalent thereto, in particular anticalins, fibronectin subdomains or individual antibody domains.

14. Method according to any of Claims 1 to 10, **characterized in that** the image molecules are carbohydrates or combinatorially synthesized compounds.

15. Method according to any of Claims 1 to 14, **characterized in that** the image molecules are modified after synthesis, in particular by means of proteolysis, phosphorylation, alkylation, glycosylation, methylation or chemical treatment.

16. Method according to Claim 15, **characterized in that** the image molecules are modified in one or more stamping processes.

17. Method according to any of Claims 1 to 16, **characterized in that** the image molecules comprise covalently or non-covalently bound anchors or chemical groups which mediate binding to the substrate surface to be printed on.

18. Method according to Claim 17, **characterized in that** the substrate surface to be printed on comprises the corresponding molecular counterpart to binding of the anchor or the substance reacting with the chemical group of the anchor.

19. Method according to Claim 18, **characterized in that** the anchor and its molecular counterpart on the substrate surface to be printed on comprise, for example, in each case complementary nucleotide sequences, or an antibody fragment and an antigen, or an S peptide and an S protein, or a biotin and a streptavidin, or a ligand and a receptor, or in each case vice versa.

20. Method according to any of Claims 1 to 19, **characterized in that** the surface of the die has topological structures in the form of a relief.

21. Method according to Claim 20, **characterized in that** the structured die comprises topological structures whose dimensions are smaller than those of the molecule spots applied to the die by means of a standard method, in particular application in drop form, and are preferably smaller than 10 µm, in particular smaller than 2 µm.

22. Method according to Claim 21, **characterized in that** molecule spots generated on the substrate are smaller than those generated on the structured die by means of a standard method, in particular application in drop form, and are preferably smaller than 10 µm, in particular smaller than 2 µm.

23. Method according to either of Claims 21 and 22, **characterized in that** repeated staggered contact printing produces an array having a higher spot density than the spot density generated on the structured die by means of a standard method, in particular application in drop form.

24. Method according to Claim 20, **characterized in that** the structured die comprises topological structures whose dimensions are smaller than the molecule spots on the differently prepared molecule array serving as a model for molecule synthesis, and are preferably smaller than 10 µm, in particular smaller than 2 µm.

25. Method according to Claim 24, **characterized in that** molecule spots generated on the substrate are smaller than the spots on the differently prepared molecule array serving as a model for molecule synthesis, and are preferably smaller than 10 µm, in particular smaller than 2 µm.

26. Method according to either of Claims 24 and 25, **characterized in that** repeated staggered contact printing generates an array having a higher density than on the differently prepared molecule array serving as a model for molecule synthesis.

27. Method according to any of Claims 1 to 26, **characterized in that** it may be used for improving the quality and simplifying the preparation of protein and, in particular, of antibody arrays or of arrays of functionally equivalent molecules, in particular anticalins, fibronectin subdomains, antibody single chains or antibody fragments.

28. Method according to Claim 27, **characterized in that** the protein or antibody arrays or arrays of functionally equivalent molecules are employed in the diagnostics of various pathogens.

## Revendications

1. Procédé de fabrication de banques de molécules disposées selon un modèle défini sur un substrat, **caractérisé par**
- la fabrication d'au moins un tampon formant matrice, qui contient une disposition parfaitement définie de molécules de matrice,
- la synthèse biochimique ou chimique in situ, en position fixe, de molécules images sur les molécules de matrice servant de modèle, sur la surface du tampon formant matrice,
- le transfert des molécules images synthétisées sur un substrat solide ou polymère, par pression par contact, tout en conservant la disposition spatiale des molécules images sur le substrat,
- la reproductibilité des étapes de synthèse et de transfert.

2. Procédé selon la revendication 1, **caractérisé en ce que** les molécules de matrice sont disposées sur le tampon formant matrice, ainsi que les molécules images sur la surface du substrat, sous forme d'une matrice de points, d'une structure circulaire, en spirale, en bandes, linéaires, ou selon une autre structure géométrique ou stochastique.

3. Procédé selon l'une des revendications 1 ou 2, **caractérisé en ce que** les banques de molécules synthétisées sur la surface du substrat peuvent être utilisées pour mettre en oeuvre des réactions parallèles de liaison.

4. Procédé selon l'une des revendications 1 à 3, **caractérisé en ce que** le tampon est au moins en partie constitué d'un matériau élastique, de préférence le polydiméthylsiloxane.

5. Procédé selon l'une des revendications 1 à 4, **caractérisé en ce qu'**on utilise deux tampons différents, ou plus, pour fabriquer une biopuce.

6. Procédé selon l'une des revendications 1 à 5, **caractérisé en ce que** les molécules de matrice sont fabriquées sur le tampon par une synthèse in situ sur les molécules servant de modèle d'une biopuce de molécules fabriquée par ailleurs.

7. Procédé selon l'une des revendications 1 à 5, **caractérisé en ce que** les molécules de matrice sont fabriquées sur le tampon par une synthèse in situ chimique ou pilotée par des champs électriques ou une lumière.

8. Procédé selon l'une des revendications 1 à 5, **caractérisé en ce que** l'application des molécules de matrice sur le tampon s'effectue par une application par gouttelettes.

9. Procédé selon l'une des revendications 1 à 5, **caractérisé en ce que** le tampon est fabriqué en un matériau transparent pour une certaine longueur d'onde, et ainsi permet, par un guidage de la lumière sélective de site, la mise en oeuvre de réactions photochimiques ou de processus optiques en champ proche pour la liaison ou la synthèse de molécules de matrice à la surface du tampon.

10. Procédé selon l'une des revendications 1 à 9, **caractérisé en ce que** le tampon est fabriqué en un matériau transparent pour une certaine longueur d'onde, et ainsi permet, par un guidage de la lumière sélective de site, une régulation de la position relative du tampon sur la surface du substrat.

11. Procédé selon l'une des revendications 1 à 10, **caractérisé en ce que** les molécules de matrice sont un ADN, un ARN ou leurs dérivés nucléase-résistants tels que le PNA ou le thioARN.

12. Procédé selon l'une des revendications 1 à 10, **caractérisé en ce que** les molécules images sont un ADN, un ARN ou des aptamères, ou leurs dérivés, en particulier nucléase-résistants, tels que le PNA ou le thioARN.

13. Procédé selon l'une des revendications 1 à 10, **caractérisé en ce que** les molécules images sont des peptides, des protéines, des anticorps ou des fragments d'anticorps, en particulier des fragments scFv, ou des molécules à fonction équivalente, en particulier des anticalines, des domaines partiels de fibronectine, ou des domaines individuels d'anticorps.

14. Procédé selon l'une des revendications 1 à 10, **caractérisé en ce que** les molécules images sont des hydrates de carbone ou des composés synthétisés par voie combinatoire.

15. Procédé selon l'une des revendications 1 à 14, **caractérisé en ce que** les molécules images sont modifiées après la synthèse, en particulier par protéolyse, phosphorylation, alkylation, glycosylation, méthylation ou traitement chimique.

16. Procédé selon la revendication 15, **caractérisé en ce que** la modification des molécules images s'effectue dans une ou plusieurs opérations de tamponnage.

17. Procédé selon l'une des revendications 1 à 16, **caractérisé en ce que** les molécules images contiennent des ancres ou des groupes chimiques liés d'une manière covalente ou non covalente, qui assurent la liaison à la surface de substrat devant être imprimée.

18. Procédé selon la revendication 17, **caractérisé en ce que** la surface de substrat devant être imprimée contient l'objet moléculaire correspondant pour la liaison de l'ancre, ou la substance réagissant avec le groupe chimique de l'ancre.

19. Procédé selon la revendication 18, **caractérisé en ce que** l'ancre et son pendant moléculaire sur la surface de substrat devant être imprimée, contiennent par exemple chacun des séquences nucléotidiques complémentaires, ou un fragment d'anticorps et un antigène, ou un peptide S et une protéine S, ou une biotine et une streptavidine, ou un ligand et un récepteur, ou inversement dans chaque cas.

20. Procédé selon l'une des revendications 1 à 19, **caractérisé en ce que** le tampon présente des structures topologiques sous forme d'un relief sur la surface.

21. Procédé selon la revendication 20, **caractérisé en ce que** le tampon structuré contient des structures topologiques qui ont des dimensions plus petites que les dépôts de molécules appliqués par un tampon par un procédé standard, en particulier une application par gouttelettes, de préférence inférieures à 10 µm et en particulier inférieures à 2 µm.

22. Procédé selon la revendication 21, **caractérisé en ce qu'**on produit sur le substrat des points de fixation de molécules plus petits que les dépôts appliqués sur le tampon structuré par un procédé standard, en particulier une application par gouttelettes, de préférence inférieures à 10 µm et en particulier inférieures à 2 µm.

23. Procédé selon l'une des revendications 21 ou 22, **caractérisé en ce qu'**on fabrique, par une impression par contact décalée et répétée, une puce ayant une densité plus élevée de points de fixation que la densité de points de fixation générée sur le tampon structuré par un procédé standard, en particulier un procédé d'application par gouttelettes.

24. Procédé selon la revendication 20, **caractérisé en ce que** le tampon structuré contient des structures topologiques qui ont des dimensions inférieures à celles du point de fixation de la molécule sur la puce de molécules fabriquée par ailleurs et servant de modèle pour la synthèse des molécules, de préférence inférieures à 10 µm, en particulier inférieures à 2 µm.

25. Procédé selon la revendication 24, **caractérisé en ce que** l'on génère sur le substrat des points de fixation de molécules plus petits que les points de fixation sur la biopuce de molécules fabriquée par ailleurs et servant de modèle à la synthèse de molécules, de préférence inférieures à 10 µm, en particulier inférieures à 2 µm.

26. Procédé selon l'une des revendications 24 ou 25, **caractérisé en ce que**, par impression par contact décalée et répétée, on fabrique une biopuce ayant une densité plus élevée que sur la puce de molécules fabriquée par ailleurs et servant de modèle à la synthèse de molécules.

27. Procédé selon l'une des revendications 1 à 26, **caractérisé en ce qu'**il peut être utilisé pour améliorer la qualité et pour simplifier la fabrication de biopuces de protéines et en particulier d'anticorps, ou de biopuces de molécules à fonction équivalente, en particulier les anticalines, les domaines partiels de fibronectine, les chaînes individuelles d'anticorps ou les fragments d'anticorps.

28. Procédé selon la revendication 27, **caractérisé en ce que** les biopuces de protéines ou d'anticorps, ou les biopuces de molécules à fonction équivalente, sont utilisées pour le diagnostic de différents microorganismes pathogènes.
